# EUROPEAN PATENT APPLICATION

(11) **EP 4 177 343 A1**
(43) Date of publication of application: **10.05.2023**
(21) Application number: 20942540.4
(22) Date of filing: 14.07.2020
(51) Int. Cl.: C12N 15/113, C12N 15/85, C12N 15/67, C12N 15/12, C12N 5/10, A61K 35/28, A61P 7/06

(54) **METHOD FOR ACTIVATING EXPRESSION OF GAMMA-GLOBIN GENE, AND COMPOSITION**

(30) Priority: 01.07.2020 CN 202010616648
(71) Applicant: Guangzhou Reforgene Medicine Co. Ltd., Guangzhou, Guangdong 510700 (CN)
(72) Inventor: LIANG, Junbin, Guangzhou, Guangdong 510700 (CN); GU, Bo, Guangzhou, Guangdong 510700 (CN); XU, Hui, Guangzhou, Guangdong 510700 (CN)
(74) Representative: Isarpatent
(86) International application number: PCT/CN2020/101936
(87) International publication number: WO 2022/000572

(57) **Abstract**

Provided is a new method for activating transcription of a gamma-globin gene. The method uses a single-stranded oligonucleotide (ssODN) containing GATA or an antisense complementary sequence TATC thereof as guidance information, and performs gene editing in a gamma-globin gene regulatory region to form a GATA-containing enhancer element, which can promote the expression of the gamma-globin gene in mature red blood cells. Hematopoietic stem cells genetically edited by the method have normal functions, can significantly improve the expression of fetal hemoglobin after being differentiated into red blood cells, and therefore can be used in clinical treatment of beta-thalassemia and sickle cell anemia.

## Description

### TECHNICAL FIELD

The present disclosure relates to the technical field of gene editing technology, and relates to a method, composition and application for activating γ-globin gene expression. The method uses a single-stranded oligonucleotide (ssODN) containing GATA or an antisense complementary sequence TATC thereof as guidance information, and performs gene editing in the γ-globin gene promoter region to form an erythroid enhancer element containing GATA, promoting γ-globin gene expression in mature erythrocytes.

### BACKGROUND

Hemoglobin (Hb for short) is a special protein that carries and transports oxygen in erythrocytes. Hemoglobin is composed of globin and heme. Hemoglobin in adults is mainly a tetramer (α2β2) composed of 2 α-globins and 2 β-globins, which becomes an adult hemoglobin (HbA). Mutations in the β-globin gene (HBB) can cause β-hemoglobin disorders (also known as β-hemoglobinopathies), including sickle cell disease (SCD) and β-thalassemia (β-thal). Sickle cell anemia is caused by point mutations in the structural gene of β-globin, resulting in the production of abnormal hemoglobins (HbS). β-thalassemia is caused by partial or complete defects in the β-globin gene expression, resulting in deficiency or absence of HbA. The reduction or absence of the globin chains of hemoglobins can lead to abnormal hemoglobin structures, the erythrocytes containing the same have reduced deformability, shortened lifespan, and may initiate *in situ* hemolysis while in the bone marrow, and may be destroyed in advance by the visceral organs such as spleen after entering the peripheral blood circulation, resulting in anemia, iron accumulation in the body and even dysplasia. Hemoglobinopathies affect millions of people around the world. Currently, approximately 330,000 children are born with hemoglobinopathies each year, which threatens human health and even life seriously. Patients with thalassemia and sickle cell disease are mainly relieved by standardized long-term blood transfusion and iron chelation therapy, which are not only incurable, but also having high safety risks. Allogeneic hematopoietic stem cell transplantation is the only treatment technology that can cure thalassemia and sickle cell disease currently. However, it is not able to be applied clinically in large scale for the limitations brought by factors such as low success rate of bone marrow matching and risk of immune rejection. Therefore, there is an urgent need to develop novel treatments which are safe and effective.

During human development, hemoglobin is not always composed of two α-globins and two β-globins. During embryonic development and shortly after birth, hemoglobin exists in the form of a tetramer (α2γ2) composed of two α-globin chains and two γ-globin chains, called fetal hemoglobin (HbF), which has a stronger oxygen affinity than HbA. With the development of fetus, the γ-globin is gradually silenced and not expressed, while the expression of β-globin gene near the same genomic site is gradually increased. About half a year after birth, the composition and proportion of hemoglobins in the blood gradually become stable, and HbF is replaced by adult hemoglobin (HbA), with only extremely low level of HbF (less than 1% of total hemoglobins) remained.

Studies have found that some populations have mutations at specific sites where the transcription of the γ-globin gene is activated, resulting in an increase in the proportion of HbF in hemoglobins, which is called hereditary persistence of fetal hemoglobin (HPFH). For example, some different types of mutations were found in the γ-globin gene promoter region, such as -114 to -102 13bp del c ., 4bp del c .-225 to -222, c .-114C>T, c .-117G>A, c .-158C>T, c .-167C>T, c .-170G>A, c .-175T>G, c .-175T>C, c .-195C>G, c .-196C>T, c .-198T>C, c .-201C>T, etc. These naturally occurring mutations increase the proportion of HbF in total hemoglobins to different extents. Clinical studies have found that in few patients with β-thalassemia or sickle cell anemia, the symptoms of anemia are reduced or relieved to a certain extent or the need for blood transfusions is reduced due to that the expression of HbF makes up the shortage of HbA resulting from the apropos presence of HPFH mutations in their genomes. Inspired by this finding, scientists continue to explore various ways to induce HbF expression to achieve the purpose of treating β-hemoglobinopathies. For example, hydroxyurea and other drugs are currently used clinically to induce HbF expression to treat β-hemoglobinopathies. However, the level of induced HbF in most patients is too low to completely improve the patient's condition.

Gene editing technology brings new hope and new methods for the treatments of genetic diseases such as hemoglobinopathies. In recent years, the breakthrough development of gene editing technology has made it possible and easier to artificially change the base sequence of specific sites in the genome. The gene editing technologies relatively well developed are ZFN (zinc finger nuclease), TALEN (transcription activator-like effector nuclease) and CRISPR (clustered regularly interspaced short palindromic repeats)/Cas system (CRISPR-Cas system). By designing a specific gene editing system, the target genomic DNA sites in a living cell is cleaved to form double-strand breaks (DSBs). Cells can use a non-homologous end joining (NHEJ) repair mechanism to repair DNA nicks, which can generate mutations randomly, such as sequence insertions, deletions, base substitutions, etc. If an artificially designed DNA donor template (donor template) is provided at the same time as gene editing, cells can use homology-directed repair (HDR) mechanism to complete the repair, thereby introducing the expected base mutation form at the target site.

Currently, there are several different approaches internationally for the therapeutic strategy of enhancing HbF expression in erythrocytes by gene editing. (1) Delete or destroy the erythroid enhancer element in the intron of BCL11A gene on human chromosome 2, and the BCL11A expression in erythrocytes is reduced, thereby the transcriptional inhibitory effect of BCL11A on the γ-globin gene is relieved. (2) Delete or destroy some sequences in the γ-globin gene promoter region to prevent the binding of transcriptional repressors, or introduce naturally occurring HPFH mutation types, such as 13 base pairs deletions (13bp del c.) at site -114 to -102. (3) Generate deletion of a large fragment of DNA of 3.5 kb to 13.6 kb, and delete the sequence containing an unknown repressor between γ-globin gene and β-globin to promote the binding of γ-globin gene to the distal LCR enhancer. The approaches have shown the effect of activating HbF expression, but the long-term efficacy or/and safety of the approaches is unclear. Therefore, new approaches need to be further developed.

### SUMMARY

The present disclosure provides a method for activating γ-globin gene transcription by gene editing. The present disclosure uses a single-stranded oligonucleotide (ssODN) containing GATA or corresponding antisense complementary sequence TATC, to generate a few base mutations (deletions, substitutions, insertions, etc.) in the proper position of the γ-globin gene promoter region by gene editing technology (such as CRISPR-Cas gene editing system). After editing, an erythroid enhancer element containing GATA is formed on the sense or antisense strand of the γ-globin gene promoter region, such as the sequence structure of NTG-N(7-8)-WGATAR or NAG-N(7-8)-WGATAR. It can promote the transcription of γ-globin gene in mature erythrocytes and increase HbF expression in erythrocytes. Herein, W is T or A; R is A or G; N is A, G, C or T, preferably C or T; N(7-8) refers to 7 to 8 random bases.

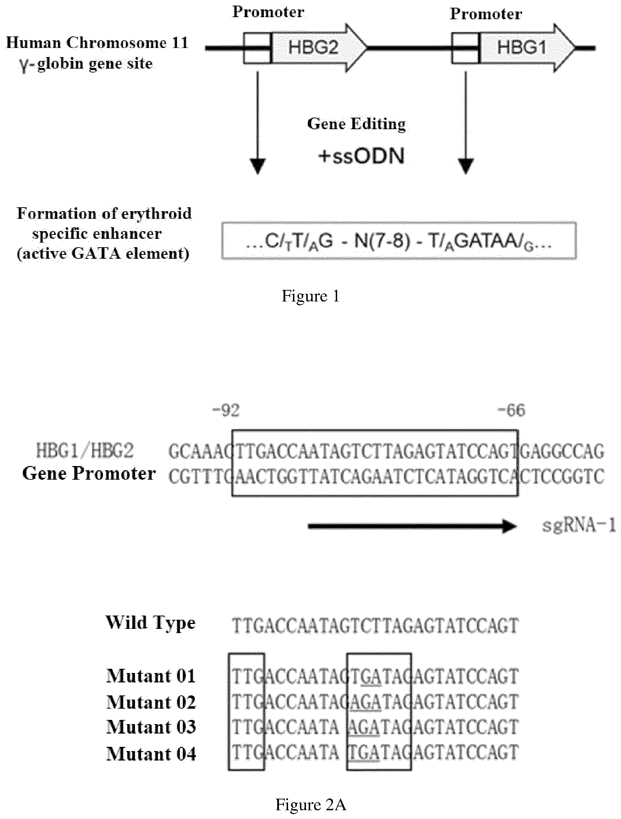

As shown in Figure 1, the principle of the present disclosure is to conduct efficient gene editing in a stem cell or a progenitor cell (such as CD34+ hematopoietic stem cell, etc.) with erythroid differentiation ability based on gene editing technology such as CRISPR-Cas, combined with the single-stranded oligonucleotide (ssODN) designed in the present disclosure,. Mutations are made in the promoter of HBG1 and HBG2 genes (located on human chromosome 11) encoding human γ-globin, and a sequence structure of NTG-N(7-8)-WGATAR or NAG-N(7-8)-WGATAR is artificially created on the sense or antisense strand of the promoter. The sequence acts as an enhancer and can recruit activating factors such as GATA1 to promote γ-globin expression after the target cell differentiated into erythrocyte.

The present disclosure provides an ssODN containing GATA or TATC sequence, which can be used for gene editing of γ-globin gene. In the present disclosure, the ssODN structure comprises a 5' homology arm, a substitution sequence and a 3' homology arm. Herein, the GATA or TATC sequence can be located at any position in the ssODN, including the substitution sequence, the 5' homology arm, the 3' homology arm, the junction of the 5' homology arm and the substitution sequence, and the junction of the 3' homology arm and the substitution sequence. The length of a unilateral homology arm could be 20 to 300 nt. In the embodiments of the present disclosure, the length of the ssODN homology arm is about 40 nt. In some embodiments of the present disclosure, the ssODN containing the GATA or TATC sequence is a sequence selected from the group consisting of SEQ ID NO: 40 to SEQ ID NO: 65. In some embodiments thereof, the 5' and 3' of the ssODN are phosphorothioate modified.

The present disclosure discloses a composition for use in γ-globin gene editing, containing ssODN, sgRNA and CRISPR-Cas nuclease. In some embodiments of the present disclosure, the site targeted by sgRNA of the gene editing system is located in the sequences shown in SEQ ID NO: 32 to SEQ ID NO: 39. In some embodiments of the present disclosure, the sgRNA is the sequence shown in SEQ ID NO: 69 to SEQ ID NO: 76. In some embodiments of the present disclosure, the Cas9 protein is a Cas 9 protein derived from *Streptococcus pyogenes.*

The disclosure also discloses an electroporation method for use in γ-globin gene editing. The method utilizes electroporation technology to efficiently transfect a human hematopoietic stem cell with the composition formed by ssODN, sgRNA, and CRISPR-Cas nuclease to mediate efficient gene editing.

The disclosure also discloses a hematopoietic stem cell. The composition comprising ssODN, sgRNA and CRISPR-Cas nuclease is transferred into the hematopoietic stem cell by electroporation method. The γ-globin gene promoter region in the cell contains active GATA element, which could increase the γ-globin gene expression during erythroid differentiation.

Compared with the prior art, the present disclosure has the following beneficial effects:

The ssODN of the present disclosure is introduced into cells together with a gene editing system (such as CRISPR-Cas, TALEN, ZFN, etc.), which guides the gene editing of the γ-globin gene promoter region, and mutate at specific sites to form GATA element with an activating effect. In some embodiments of the present disclosure, the ssODN and the CRISPR-Cas gene editing system are introduced into human hematopoietic stem cells by electroporation technology, and efficient gene editing is achieved in the γ-globin gene promoter region. Mutations are introduced at specific sites to form the expected erythroid enhancer element containing GATA. After the hematopoietic stem cells differentiate into erythrocytes, the γ-globin gene expression significantly increases and the proportion of HbF in hemoglobin significantly increases.

### BRIEF DESCRIPTION OF THE DRAWINGS

Figure 1 illustrates the technical principle of the present disclosure.
Figure 2A shows that the gene editing targeting region is the wild-type sequence between -92 and -66 of HBG1 and HBG2 genes, also shows the position of the gRNA targeting sequence used and the mutated sequence expected after gene editing.
Figure 2B shows that the gene editing targeting region is the wild-type sequence between -129 and -98 of HBG1 and HBG2 genes, also shows the position of the gRNA targeting sequence used and the mutated sequence expected after gene editing.
Figure 2C shows that the gene editing targeting region is the wild-type sequence between -175 and -153 of HBG1 and HBG2 genes, also shows the position of the gRNA targeting sequence used and the mutated sequence expected after gene editing.
Figure 2D shows that the gene editing targeting region is the wild-type sequence between -192 and -160 of HBG1 and HBG2 genes, also shows the position of the gRNA targeting sequence used and the mutated sequence expected after gene editing.
Figure 2E shows that the gene editing targeting region is the wild-type sequence between -428 and -406 of HBG1 gene and -432 and -410 of HBG2 gene, also shows the position of the gRNA targeting sequence used and the mutated sequence expected after gene editing.
Figure 3 shows the editing efficiency data of HBG1 and HBG2 genomes by transfection of CRISPR-Cas plasmids in 293T cells. The data comes from the INDEL detection results of genomic DNA four days after the transfection of CRISPR-Cas plasmids in 293T cells using Lipofectamine 2000^{™}. The figure shows the percentage of total indel mutations (% of total mutations).
Figure 4 shows the editing efficiency data of HBG2 genome by simultaneous transfection of CRISPR-Cas plasmid and ssODN in 293T cells. The data comes from the INDEL detection results of HBG2 four days after the transfection of CRISPR-Cas plasmid and ssODN in 293T cells using Lipofectamine 2000^{™}.
Figure 5 shows the editing efficiency data of HBG2 genome by simultaneous transfection of CRISPR-Cas plasmid and ssODN in K562 cells. The data comes from the INDEL detection results of HBG2 four days after the transfection of CRISPR-Cas plasmid and ssODN in K562 cells using Lonza 4D electroporation.
Figure 6 shows the editing efficiency data of HBG2 genome after transduction of ribonucleoprotein (RNP) and ssODN in K562 cells by electroporation. The data comes from the INDEL detection results of HBG2 four days after the transduction of ssODN and CRISPR-Cas/sgRNA RNP in K562 cells using electroporation.
Figures 7A-7C shows the editing efficiency data of HBG2 gene after transduction of RNP and ssODN in mobilized peripheral blood CD34+ cells (mPBSCs) by electroporation. The data comes from the INDEL detection results of genomic DNA 5 days after the induction of erythroid differentiation followed by the transduction of ssODN and CRISPR-Cas/sgRNA RNP in mPBSCs by electroporation. Figures 7A and 7B are Sanger sequencing maps, and Figure 7C is the INDEL ratio result after analysis by synthego software.
Figure 8 shows the γ-globin mRNA transcription data after gene editing of mobilized peripheral blood CD34+ cells (mPBSCs). The data comes from the qRT-PCR detection result of mRNA 18 days after the induction of erythroid differentiation followed by the transduction of ssODN and CRISPR-Cas/sgRNA RNP in mPBSCs by electroporation. The expression level of GAPDH is set as the internal reference to the γ-globin mRNA expression in the figure, and the γ-globin mRNA expression was normalized with the γ-globin mRNA expression in cells without editing as a reference, n=3.
Figure 9 shows the HbF expression data after gene editing of mobilized peripheral blood CD34+ cells (mPBSCs). The data comes from HPLC detection result of hemoglobin after 18 days after the induction of erythroid differentiation followed by the transduction of ssODN and CRISPR-Cas/sgRNA RNP in mPBSCs by electroporation. HbF expression in the figure is the percentage of HbF in total hemoglobin.

### DETAILED DESCRIPTION OF THE EMBODIMENTS

In order to facilitate understanding of the present disclosure, the present disclosure will be described more comprehensively with reference to the related drawings as follows. Preferred embodiments of the disclosure are shown in the drawings but the present disclosure may be embodied in many different forms and is not limited thereto. On the contrary, the purpose of providing these embodiments is to understand the present disclosure thoroughly and comprehensively.

Unless otherwise defined, all technical and scientific terms used herein have the same meaning as commonly understood by the skilled in the art. The terms used herein in the description of the present disclosure are for the purpose of describing specific embodiments only and are not intended to limit the present disclosure. As used herein, the term "and/or" includes any and all combinations of one or more of the associated listed items.

The raw materials used in the following examples are all commercially available.

The disclosure provides by using of gene editing technology (such as CRISPR-Cas-mediated gene editing technology), combined with the single-stranded oligonucleotides (ssODN) designed by the present disclosure, performing efficient gene editing on stem cells and progenitor cells with erythroid differentiation ability such as CD34+ hematopoietic stem cells. Mutations are made in the promoter of HBG1 and HBG2 genes (located on human chromosome 11) encoding human γ-globin, and a sequence structure of NTG-N(7-8)-WGATAR or NAG-N(7-8)-WGATAR is artificially created on the sense or antisense strand of the promoter (Figure 1). The sequence acts as an enhancer and can recruit activating factors such as GATA1 to promote γ-globin expression after the target cells differentiated into erythrocytes.

GATA-1 is a transcriptional regulator that is specifically and highly expressed in the erythroid system and plays an important role in the process of erythroid differentiation. Genes that are specifically highly expressed in erythrocytes, such as α- and β-globin and heme biosynthetic enzymes, are directly activated by GATA-1 transcription. As a transcription factor, GATA-1 selectively binds to WGATAR sequence element on chromatin DNA (wherein W represents T or A, R represents A or G, and can also be represented by T/A(GATA)A/G; the antisense complementary sequence is YTATCW, wherein Y represents T or C, and can also be represented by T/C(TATC)T/A), for example, multiple GATA sites have also been found in the regulatory region of the human β-globin gene. Notably, not all genes containing GATA sequence would be activated by GATA-1. This is because GATA sequence is widely distributed in the genome and often act with a need to form a certain form of sequence combination (element combination) with upstream and downstream sequences (*cis* elements). For example, there are cis-regulatory elements such as CACCC or GC box near the GATA sequence, which usually recruit GATA-1 to bind together to the transcription factor EKLF. For example, there is a E-box element (CAGNTG, wherein N represents any base) or half E-box element (NTG) near GATA sequence, which recruits GATA-1 to bind together to the transcription fact TAL1. ChIP-seq studies on the binding sites of GATA-1 and TAL1 at the genome level showed that about three-quarters of the GATA-1/TAL1 co-binding sites have a obvious sequence regularity, that there is often a half E-box element (NTG) which is 7-8 bases upstream of the WGATA sequence, which can be represented by NTG-N(7-8)-WGATA. Experiments have shown that this sequence is an active GATA element that promotes gene expression in erythrocytes. For example, the +58 enhancer in the BCL11A intron contains the CTG-N(7)-TGATAA sequence, and the disruption of the GATA motif in this sequence can lead to a significantly reduction of BCL11A expression in erythrocytes.

The sequences of 1400 bases upstream of the transcription start site of HBG1 and HBG2 genes have an extremely high similarity and are considered to be the promoter region. The inventor of the present disclosure has found from studies that the promoter region of the γ-globin itself contains multiple GATA sequence (or TATC) and a large number of TG sequence, but γ-globin is hardly expressed in mature erythrocytes. The inventor of the present disclosure has found through profound research that GATA (or TATC) and TG sequences of the γ-globin gene promoter do not form effective active GATA elements in position and direction (such as TG-N (7-8) -WGATAR), and therefore cannot recruit transcription activators such as GATA-1/TAL1 that are highly expressed in mature erythrocytes to bind (co-bind) to the promoter region of the γ-globin gene, so γ-globin is silenced and is hardly expressed in mature erythrocytes.

Through profound research, the inventor of the present disclosure found that there are some sites in the promoter regions of HBG1 and HBG2, the sequences of the sense or antisense strands of which can form the sequence structure of NTG-N(7-8)-WGATAR or NAG-N(7-8)-WGATAR by changes (such as substitutions, deletions, insertions, etc.) of a few bases. The inventor of the present disclosure consider that it is possible to edit a certain proportion of the genome to an expected sequence structure if a gene editing system is used to cleave the sites to form DSBs, by adding a donor DNA template (such as a single-stranded oligonucleotide ssODN) to guide the sites for HDR repair. Finally, the γ-globin gene promoter region, can act as an enhancer to recruit highly-expressed transcription activators in mature erythrocytes such as GATA-1/TAL1, etc. to bind (co-bind) to the promoter region of the γ-globin gene due to the formation of the sequence such as NTG-N(7-8)-WGATAR or NAG-N(7-8)-WGATAR, thereby promotes the high expression of γ-globin in mature erythrocytes.

Gene editing can be divided into two categories in terms of purposes. One is to destroy the sequence or structure of the target site, which is called knock out. The purpose of another type is to introduce specific sequence mutation at the target site, which is called knock in. To introduce specific sequence mutations, the most common method is to introduce a DNA donor template at the same time, and cells can use the mechanism of homologous recombination to replace the sequence in the template in the genome to achieve knock-in. DNA templates for HDR can be plasmid DNA, linearized double-stranded DNA, AAV-delivered DNA, or single-stranded oligonucleotides (ssODN). ssODN is used more and more in gene editing, as it has high efficiency and is easy to synthesis, which is particularly suitable for gene editing in cultured cells *in vitro.*

The ssODN structure contains a 5' homology arm, a substitution sequence, and a 3' homology arm. Homology arms are sequences that are homologous to the DNA regions flanking the target site intended to be edited, and are used to locate the target site on the chromosome. The corresponding sequences of the 5' homology arm and the 3' homology arm on the chromosome are usually discontinuous in position, with a certain space among them, such as an spacer of 1-20 nucleotides, and these spacer sequences are the targets (intended-editing sequence) of gene editing, i.e., the sequence that is desired to be substituted by gene editing. The "substitution sequence" between the 3' terminus of the 5' homology arm and the 5' terminus of the 3' homology arm on the ssODN is the desired result of gene editing. It is able to perform homologous recombination repair while inducing gene editing by adding ssODN while performing gene editing. As a result of the repair, the intended-editing sequence in the cell genome is repaired into a substitution sequence. The length of the substitution sequence in the ssODN can be shorter than that of the intended-editing sequence, in which case the result of gene editing is the deletion or/and substitution of the intended-editing sequence in the original genome. The length of the substitution sequence in the ssODN can be 0 base, in which case the intended-editing sequence on the genome is deleted. The length of the substitution sequence in the ssODN may be longer than that of the intended-editing sequence, in which case the result of gene editing is that the intended-editing sequence is replaced or/and sequence insertion.

The present disclosure is suitable for gene editing systems mediated by site-specific nucleases such as "CRISPR-Cas", TALEN and ZFN. "CRISPR-Cas" is a gene editing technology, including but not limited to various natural or artificially designed CRISPR-Cas systems, such as the CRISPR-Cas9 system, CRISPR-Cas 12 system, etc. The working principle of CRISPR-Cas9 is that crRNA (CRISPR-derived RNA) combines with tracrRNA (trans-activating RNA) through base pairing to form a tracrRNA/crRNA complex, which guides the nuclease Cas9 protein to cleave double-stranded DNA at the sequence target sites paired with crRNA. The role of tracrRNA and crRNA can also be substituted by an artificially synthesized sgRNA with a guiding role. When using other CRISPR-Cas systems, it is necessary to design the corresponding sgRNA or crRNA. When using systems such as TALEN or ZFN, the corresponding TALEN or ZFN nuclease needs to be designed according to the editing site disclosed in the present disclosure.

### Example 1: Screening of ssODN and CRISPR-Cas systems for the introduction of an active GATA element into the γ-globin gene promoter

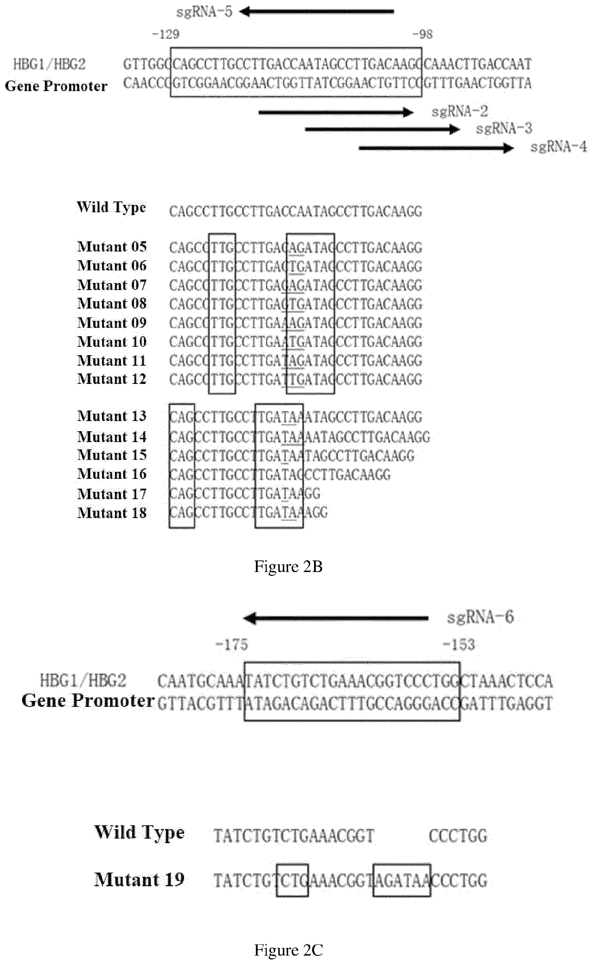

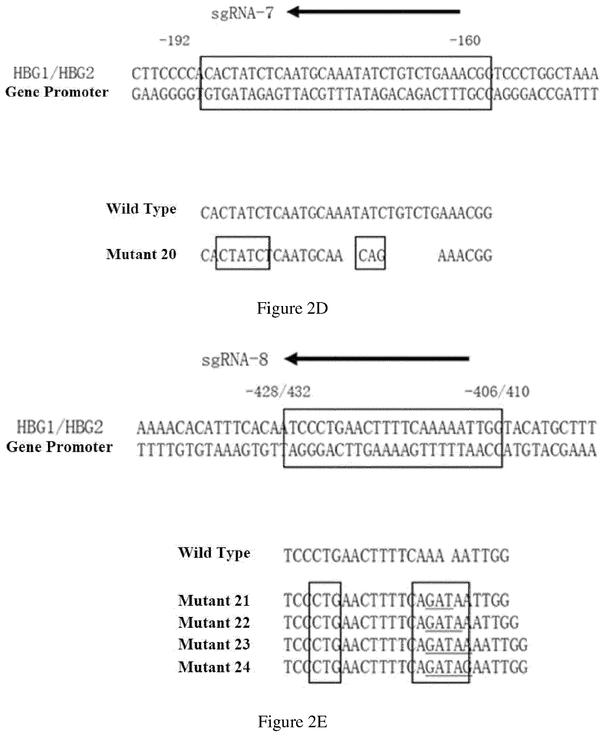

By analyzing the sequences (SEQ ID NO: 1 and SEQ ID NO: 2) within about 1.4 kb upstream of the transcription start sites of HBG1 and HBG2, the inventor found that the sequences of many segments satisfy that forming the sequence structure of NTG-N(7-8)-WGATAR or NAG-N(7-8)-WGATAR by changes of a few bases such as substitution, deletion, insertion, etc.. By analyzing whether there is an NGG (PAM recognized by spCas9) sequence near the target mutant base in these segments and whether the cleavage site is on target of the target sequence to be edited, five candidate targeting regions most likely to achieve efficient gene editing were finally obtained (Figures 2A to 2E). According to the distance from the transcription start site, they are: Region 1, between -92 to -66 of HBG1 and HBG2 promoters (Figure 2A); Region 2, between -129 to -98 of HBG1 and HBG2 promoters (Figure 2B); Region 3, between -175 and -153 of HBG1 and HBG2 promoters (Figure 2C); Region 4, between -192 and -160 of HBG1 and HBG2 genes (Figure 2D); Region 5, between -428 to -406 of HBG1 gene and -432 to -410 of HBG2 gene (Figure 2E).

According to the sequence features of the five candidate regions and the predicted cleavage site position of the CRISPR-Cas9 gene editing system, the suitable mutation target types in each region is obtained by analyzing, as is shown in Table 1 (SEQ ID NO: 3 to SEQ ID NO: 31). The underline herein indicates the base that needs to be substituted, which is the desired result after gene editing. In the present disclosure, the length of the substitution base is 0-6 bases, and the effect achieved is deletion, substitution, insertion, or a combination of deletion, substitution, and insertion.

**Table 1 Wild-type sequences of HBG1 and HBG2 promoters and sequences obtained after editing**

| SEQ ID | Number | Target site and edited sequence (5' -3') |
|---|---|---|
| SEQ ID NO:3 | Region 1, wild-type | TTGACCAATAGTCTTAGAGTATCCAGT |
| SEQ ID NO:4 | Mutant_01 | TTGACCAATAGTGATAGAGTATCCAGT |
| SEQ ID NO:5 | Mutant_02 | TTGACCAATAGAGATAGAGTATCCAGT |
| SEQ ID NO:6 | Mutant_03 | TTGACCAATAAGATAGAGTATCCAGT |
| SEQ ID NO:7 | Mutant_04 | TTGACCAATATGATAGAGTATCCAGT |
| SEQ ID NO:8 | Region 2, wild-type | CAGCCTTGCCTTGACCAATAGCCTTGACAAGG |
| SEQ ID NO:9 | Mutant_05 | CAGCCTTGCCTTGACAGATAGCCTTGACAAGG |
| SEQ ID NO:10 | Mutant_06 | CAGCCTTGCCTTGACTGATAGCCTTGACAAGG |
| SEQ ID NO:11 | Mutant_07 | CAGCCTTGCCTTGAGAGATAGCCTTGACAAGG |
| SEQ ID NO:12 | Mutant_08 | CAGCCTTGCCTTGAGTGATAGCCTTGACAAGG |
| SEQ ID NO:13 | Mutant_09 | CAGCCTTGCCTTGAAAGATAGCCTTGACAAGG |
| SEQ ID NO:14 | Mutant_10 | CAGCCTTGCCTTGAATGATAGCCTTGACAAGG |
| SEQ ID NO:15 | Mutant_11 | CAGCCTTGCCTTGATAGATAGCCTTGACAAGG |
| SEQ ID NO:16 | Mutant_12 | CAGCCTTGCCTTGATTGATAGCCTTGACAAGG |
| SEQ ID NO:17 | Mutant_13 | CAGCCTTGCCTTGATAAATAGCCTTGACAAGG |
| SEQ ID NO:18 | Mutant_14 | CAGCCTTGCCTTGATAAAATAGCCTTGACAAGG |
| SEQ ID NO:19 | Mutant_15 | CAGCCTTGCCTTGATAATAGCCTTGACAAGG |
| SEQ ID NO:20 | Mutant_16 | CAGCCTTGCCTTGATAGCCTTGACAAGG |
| SEQ ID NO:21 | Mutant_17 | CAGCCTTGCCTTGATAAGG |
| SEQ ID NO:22 | Mutant_18 | CAGCCTTGCCTTGATAAAGG |
| SEQ ID NO:23 | Region 3, wild-type | TATCTGTCTGAAACGGTCCCTGG |
| SEQ ID NO:24 | Mutant_19 | TATCTGTCTGAAACGGTAGATAACCCTGG |
| SEQ ID NO:25 | Region 4, wild-type | CACTATCTCAATGCAAATATCTGTCTGAAACGG |
| SEQ ID NO:26 | Mutant_20 | CACTATCTCAATGCAACAGAAACGG |
| SEQ ID NO:27 | Region 5, wild-type | TCCCTGAACTTTTCAAAAATTGG |
| SEQ ID NO:28 | Mutant_21 | TCCCTGAACTTTTCAGATAATTGG |
| SEQ ID NO:29 | Mutant_22 | TCCCTGAACTTTTCAGATAAATTGG |
| SEQ ID NO:30 | Mutant_23 | TCCCTGAACTTTTCAGATAAAATTGG |
| SEQ ID NO:31 | Mutant_24 | TCCCTGAACTTTTCAGATAGAATTGG |

In order to cleave the DNA double-strand near the editing site of regions shown in Table 1 to form DSB, the spCas9 CRISPR-Cas system with higher cleavage efficiency (derived from *Streptococcus pyogenes*) is selected in the present disclosure to analyze the target sites with higher cleavage efficiency. Site_1 to site_8 were selected as candidate target sites. The identified target site of DNA sequences and PAM sequences (NGG) are shown in Table 2 (SEQ ID NO: 32 to SEQ ID NO: 39).

**Table 2 Target sites recognized by sgRNAs on HBG1 and HBG2 promoters**

| SEQ ID | Site | sgRNA recognition site and PAM (5'-3') | DNA strand |
|---|---|---|---|
| SEQ ID NO:32 | site_1 | ACTGGATACTCTAAGACTAT TGG | antisense strand |
| SEQ ID NO:33 | site_2 | CTTGTCAAGGCTATTGGTCA AGG | antisense strand |
| SEQ ID NO:34 | site_3 | GTTTGCCTTGTCAAGGCTAT TGG | antisense strand |
| SEQ ID NO:35 | site_4 | TGGTCAAGTTTGCCTTGTCA AGG | antisense strand |
| SEQ ID NO:36 | site_5 | CTTGACCAATAGCCTTGACA AGG | sense strand |
| SEQ ID NO:37 | site_6 | TATCTGTCTGAAACGGTCCC TGG | sense strand |
| SEQ ID NO:38 | site_7 | ATGCAAATATCTGTCTGAAA CGG | sense strand |
| SEQ ID NO:39 | site_8 | TCCCTGAACTTTTCAAAAAT TGG | sense strand |

According to the expected mutation type shown in Table 1, and the DNA strand (sense strand or antisense strand) recognized by sgRNA in Table 2, the corresponding ssODN is designed to guide gene editing and repair (Table 3, SEQ ID NO: 40 to SEQ ID NO: 65). In the present disclosure, the ssODN structure comprises a 5' homology arm, a substitution sequence and a 3' homology arm. Herein, the GATA or TATC sequence can be located at any position on the ssODN, including the substitution sequence, the 5' homology arm, the 3' homology arm, the junction of the 5' homology arm and the substitution sequence, and the junction of the 3' homology arm and the substitution sequence. The homology arms on both sides of the ssODN can be symmetric or asymmetric (the lengths on both sides are different). The ssODN with symmetric homology arms is uniformly used for experiments in the present disclosure for the convenience of system comparison. The length of the homology arms at both sides of ssODN is 20 to 300 nt. The ssODN with a homology arm of approximately 40 nt on both sides is used to illustrate in the examples of the present disclosure for the convenience of system comparison. The ssODN can be the sense strand or the antisense strand of the edited region DNA (the homology arm sequence is the same with the corresponding sense strand or antisense strand). In the present disclosure, the DNA strand with the same sgRNA recognition site is uniformly selected by ssODN as the ssODN template sequence. Except for the sequences of homology arms on both sides, the substitution base of the sequence of ssODN can be 0, 1, 2, 3, 4, 5 or 6 bases, and the effect achieved is deletion, substitution, insertion, or a combination of deletion, substitution, and insertion. The effect achieved can be to change more than 1 base on the genome, such as deletion or substitution of 1-20 bases. In the sequences as shown in Table 3 (SEQ ID NO: 40 to SEQ ID NO: 65), the underlined bases are homology arms on both sides with a length of about 40 nt, the non-underlined bases between the homology arms are substitution bases, wherein ssODN_16 has 0 substitution base between the homology arms on both sides. The first three nucleotides at both terminuses of ssODN were modified with phosphorothioate to enhance the stability of ssODN and improve its activity in gene editing.

**Table 3 ssODN sequence and the matched gRNA number**

| SEQ ID | ssODN | ssODN sequence (5'- 3') | Applicable area |
|---|---|---|---|
| SEQ ID NO:40 | ssODN_1 | CCTAGCCAGCCGCCGGCCCCTGGCCTCACTGGATACT CTATCACTATTGGTCAAGTTTGCCTTGTCAAGGCTAT TGGTCAAGGC | Region 1 |
| SEQ ID NO:41 | ssODN_2 | CCTAGCCAGCCGCCGGCCCCTGGCCTCACTGGATACT CTATCTCTATTGGTCAAGTTTGCCTTGTCAAGGCTATT GGTCAAGGC | Region 1 |
| SEQ ID NO:42 | ssODN_3 | CCTAGCCAGCCGCCGGCCCCTGGCCTCACTGGATACT CTATCTTATTGGTCAAGTTTGCCTTGTCAAGGCTATTG GTCAAGGC | Region 1 |
| SEQ ID NO:43 | ssODN_4 | CCTAGCCAGCCGCCGGCCCCTGGCCTCACTGGATACT CTATCATATTGGTCAAGTTTGCCTTGTCAAGGCTATT GGTCAAGGC | Region 1 |
| SEQ ID NO:44 | ssODN_5 | CTCTAAGACTATTGGTCAAGTTTGCCTTGTCAAGGCT ATCTGTCAAGGCAAGGCTGGCCAACCCATGGGTGGA GTTTAGCCA | Region 2 |
| SEQ ID NO:45 | ssODN_6 | CTCTAAGACTATTGGTCAAGTTTGCCTTGTCAAGGCT ATCAGTCAAGGCAAGGCTGGCCAACCCATGGGTGGA GTTTAGCCA | Region 2 |
| SEQ ID NO:46 | ssODN_7 | CTCTAAGACTATTGGTCAAGTTTGCCTTGTCAAGGCT ATCTCTCAAGGCAAGGCTGGCCAACCCATGGGTGGA GTTTAGCCA | Region 2 |
| SEQ ID NO:47 | ssODN_8 | CTCTAAGACTATTGGTCAAGTTTGCCTTGTCAAGGCT ATCACTCAAGGCAAGGCTGGCCAACCCATGGGTGGA GTTTAGCCA | Region 2 |
| SEQ ID NO:48 | ssODN_9 | CTCTAAGACTATTGGTCAAGTTTGCCTTGTCAAGGCT ATCTTTCAAGGCAAGGCTGGCCAACCCATGGGTGGA GTTTAGCCA | Region 2 |
| SEQ ID NO:49 | ssODN_10 | CTCTAAGACTATTGGTCAAGTTTGCCTTGTCAAGGCT ATCATTCAAGGCAAGGCTGGCCAACCCATGGGTGGA GTTTAGCCA | Region 2 |
| SEQ ID NO:50 | ssODN_11 | CTCTAAGACTATTGGTCAAGTTTGCCTTGTCAAGGCT ATCTATCAAGGCAAGGCTGGCCAACCCATGGGTGGA GTTTAGCCA | Region 2 |
| SEQ ID NO:51 | ssODN_12 | CTCTAAGACTATTGGTCAAGTTTGCCTTGTCAAGGCT ATCAATCAAGGCAAGGCTGGCCAACCCATGGGTGGA GTTTAGCCA | Region 2 |
| SEQ ID NO:52 | ssODN_13 | CTCTAAGACTATTGGTCAAGTTTGCCTTGTCAAGGCT ATTTATCAAGGCAAGGCTGGCCAACCCATGGGTGGA GTTTAGCCA | Region 2 |
| SEQ ID NO:53 | ssODN_14 | CTCTAAGACTATTGGTCAAGTTTGCCTTGTCAAGGCT ATTTTATCAAGGCAAGGCTGGCCAACCCATGGGTGG AGTTTAGCCA | Region 2 |
| SEQ ID NO:54 | ssODN_15 | CTCTAAGACTATTGGTCAAGTTTGCCTTGTCAAGGCT ATTATCAAGGCAAGGCTGGCCAACCCATGGGTGGAG TTTAGCCA | Region 2 |
| SEQ ID NO:55 | ssODN_16 | CTCTAAGACTATTGGTCAAGTTTGCCTTGTCAAGGCT | Region 2 |
| | | A TCAAGGCAAGGCTGGCCAACCCATGGGTGGAGTTTA GCCA | |
| SEQ ID NO:56 | ssODN_17 | CCTCACTGGATACTCTAAGACTATTGGTCAAGTTTGC CTTATCAAGGCAAGGCTGGCCAACCCATGGGTGGAG TTTAGCCA | Region 2 |
| SEQ ID NO:57 | ssODN_18 | CCTCACTGGATACTCTAAGACTATTGGTCAAGTTTGC CTTTATCAAGGCAAGGCTGGCCAACCCATGGGTGGA GTTTAGCCA | Region 2 |
| SEQ ID NO:58 | ssODN_19 | TGGCTAAACTCCACCCATGGGTTGGCCAGCCTTGCCT TGATTAAGGCAAACTTGACCAATAGTCTTAGAGTATCC AGTGAGG | Region 2 |
| SEQ ID NO:59 | ssODN_20 | TGGCTAAACTCCACCCATGGGTTGGCCAGCCTTGCCT TGATAAAGGCAAACTTGACCAATAGTCTTAGAGTATC CAGTGAGG | Region 2 |
| SEQ ID NO: 60 | ssODN_21 | TCCCCACACTATCTCAATGCAAATATCTGTCTGAAAC GGTAGATAACCCTGGCTAAACTCCACCCATGGGTTGG CCAGCCTTGCCT | Region 3 |
| SEQ ID NO:61 | ssODN_22 | TATCCTCTTGGGGGCCCCTTCCCCACACTATCTCAAT GCAACAGAAACGGTCCCTGGCTAAACTCCACCCATG GGTTGGCCAGC | Region 4 |
| SEQ ID NO: 62 | ssODN_23 | CCTATGCCTAAAACACATTTCACAATCCCTGAACTTT TCAGATAATTGGTACATGCTTTAACTTTAAACTACAG GCCTCACTG | Region 5 |
| SEQ ID NO: 63 | ssODN_24 | CCTATGCCTAAAACACATTTCACAATCCCTGAACTTT TCAGATAAATTGGTACATGCTTTAACTTTAAACTACA GGCCTCACTG | Region 5 |
| SEQ ID NO: 64 | ssODN_25 | CCTATGCCTAAAACACATTTCACAATCCCTGAACTTT TCAGATAAAATTGGTACATGCTTTAACTTTAAACTAC AGGCCTCACTG | Region 5 |
| SEQ ID NO: 65 | ssODN_26 | CCTATGCCTAAAACACATTTCACAATCCCTGAACTTT TCAGATAGAATTGGTACATGCTTTAACTTTAAACTAC AGGCCTCACTG | Region 5 |

The sgRNA-spCas9 vector (PX459, pSpCas9(BB)-2A-Puro) is used to express the sgRNA corresponding to the target site in Table 2. The vector expresses the spCas9 protein and can express sgRNA with a length of 100 nt. The 5' terminus of the sgRNA is a guide sequence of 20 nt, and the latter is a general sgRNA backbone sequence of 80 nt (SEQ ID NO: 66, GUUUUAGAGCUAGAAAUAGCAAGUUAAAAUAAGGCUAGUCCGUUAUCAACUUGA AAAA GUGGCACCGAGUCGGUGCUUUU). The method of cloning sgRNA is: synthesize the corresponding guide strand sequence oligo (same as the 20nt target site sequence in Table 2) of the sgRNA and complementary strand oligo (cacc is added to the 5'-terminus of the guide strand sequence, if the first nucleotide at the 5'-terminus of the guide strand is not guanine G, then caccG is added to the 5'-terminus of the guide strand; cacc is added to the 5'-terminus of the complementary strand; if a G is added to the 5'-terminus of the guide strand, then a C is added to the 3'-terminus of the complementary strand for complementary pairing). The two oligos are mixed in equal amounts, subjected to heat deformation and annealed at 95°C, and then ligated into the PX459 vector digested with the restriction enzyme Bbs I. The success of cloning was identified by colony PCR and Sanger sequencing. The PCR was performed using SEQ ID NO: 67 (5'-3': GGACTATCATATGCTTACCGTAAC) and SEQ ID NO: 68 (5'-3': GGCGGGCCATTTACCGTAAG) as primers. After successfully cloning, 8 plasmids were obtained, expressing sgRNAs from SEQ ID NO: 69 to SEQ ID NO: 76, respectively (Table 4).

**Table 4 sgRNAs expressed by PX459 vector**

| SEQ ID | sgRNA | sgRNA sequence (5'- 3') |
|---|---|---|
| SEQ ID NO: 69 | sgRNA-1 | ACUGGAUACUCUAAGACUAU GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:70 | sgRNA-2 | CUUGUCAAGGCUAUUGGUCA GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:71 | sgRNA-3 | GUUUGCCUUGUCAAGGCUAU GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:72 | sgRNA-4 | UGGUCAAGUUUGCCUUGUCA GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:73 | sgRNA-5 | CUUGACCAAUAGCCUUGACA GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:74 | sgRNA-6 | UAUCUGUCUGAAACGGUCCC GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:75 | sgRNA-7 | AUGCAAAUAUCUGUCUGAAA GUUUUAGAGCUAGAAAUAGCAAGUUAAAAUAAGGCUAGUCCGUUAU CAACUUGAAAAAGUGGCACCGAGUCGGUGCUUUU |
| SEQ ID NO:76 | sgRNA-8 | UCCCUGAACUUUUCAAAAAU GUUUUAGAGCUAGAAAUAGCAAGUUAAAAUAAGGCUAGUCCGUUAU CAACUUGAAAAAGUGGCACCGAGUCGGUGCUUUU |

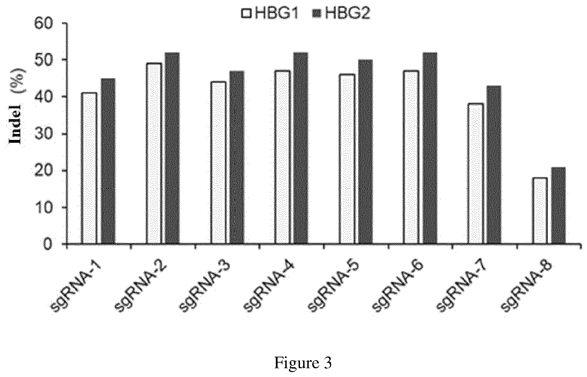

The 8 plasmids expressed were transfected into 293T cells, and the method was referred to the method of Lipofectamine 2000^{™} kit. The transfected cells were cultured for 4 days, and the cells were collected to extract genomic DNA. SEQ ID NO: 77 and SEQ ID NO: 78, and SEQ ID NO: 79 and SEQ ID NO: 78 were used respectively, to amplify the fragments of HBG1 and HBG2 promoter gene by KOD Plus high-fidelity enzyme PCR method. The resulting PCR products were purified and sequenced using SEQ ID NO: 77 and SEQ ID NO: 79, respectively. The files obtained from the sequencing results were analyzed by synthego software for gene editing efficiency. As shown in Figure 3, except for sgRNA-8 with a relatively low editing efficiency (about 20%), the overall mutation (indel) efficiency of editing in other groups is about 40-50%.

**Table 5 PCR and Sanger sequencing primers**

| SEQ ID | site | sgRNA recognition site and PAM (5'- 3') |
|---|---|---|
| SEQ ID NO:77 | HBG1_F | TACTGCGCTGAAACTGTGGCTT |
| SEQ ID NO:78 | HBG1/2_R | CTTCCCAGGGTTTCTCCTCCAG |
| SEQ ID NO:79 | HBG2_F | TGCACTGAAACTGTTGCTTTATAGGA |

### Example 2: Co-transfection of ssODN and sgRNA-spCas9 vector into 293T cells for gene editing

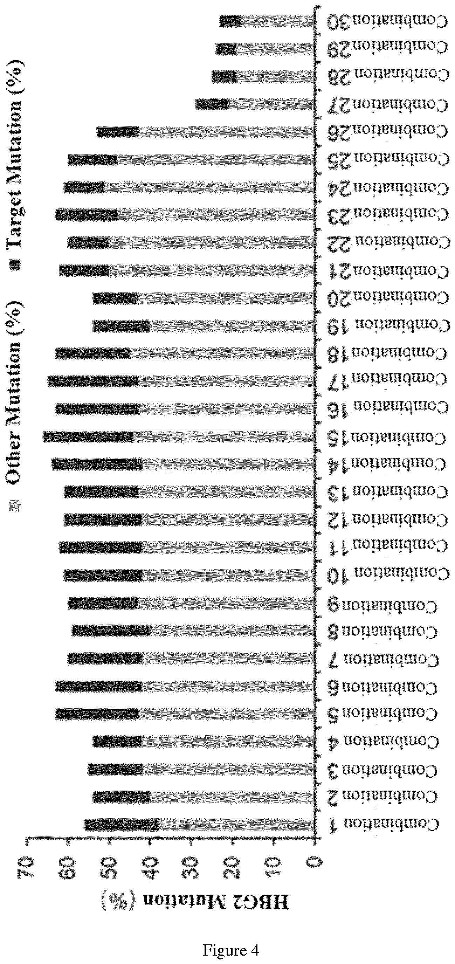

The eight sgRNA-spCas9 vectors were co-transfected with appropriate ssODN into 293T cells to test whether the addition of ssODN could induce gene editing to form the expected mutation type (occurrence of HDR repair). According to the position of the sgRNA cleavage point, the 8 sgRNA-spCas9 vectors were combined according to the sgRNA + ssODN combination method shown in Table 6 to form 30 combinations, respectively. Using Lipofectamine 2000^{™} reagent, ssODN was added while transfecting the plasmid, and the ssODN and the plasmid were delivered to the cells together. The transfected cells were cultured for 4 days, and the cells were collected to extract genomic DNA. Since the gene editing efficiency of HBG1 and HBG2 is almost positive correlated, and the gene editing efficiency of HBG2 is always slightly higher than that of HBG1 (Figure 3), only the gene editing efficiency of HBG2 genome was selected for comparison in subsequent experiments.The fragment of HBG2 promoter gene was amplified by PCR, purified and sequencing according to the methods described in Example 1. The sequencing results analyzed by synthego software for gene editing efficiency, show that the addition of ssODN and the co-transfection with the sgRNA-spCas9 vector could improve the editing efficiency, and most groups induced about 10-20% of the gene editing to form the expected mutation type at the same time (Figure 4).

**Table 6 Co-transfection combinations of ssODN with sgRNA-spCas9 vector**

| Combination | Transfection complex | Combination | Transfection complex |
|---|---|---|---|
| Combination 1 | sgRNA-1 + ssODN_1 | Combination 16 | sgRNA-2 + ssODN_16 |
| Combination 2 | sgRNA-1 + ssODN_2 | Combination 17 | sgRNA-2 + ssODN_17 |
| Combination 3 | sgRNA-1 + ssODN_3 | Combination 18 | sgRNA-2 + ssODN_18 |
| Combination 4 | sgRNA-1 + ssODN_4 | Combination 19 | sgRNA-3 + ssODN_17 |
| Combination 5 | sgRNA-2 + ssODN_5 | Combination 20 | sgRNA-3 + ssODN_18 |
| Combination 6 | sgRNA-2 + ssODN_6 | Combination 21 | sgRNA-4 + ssODN_17 |
| Combination 7 | sgRNA-2 + ssODN_7 | Combination 22 | sgRNA-4 + ssODN_18 |
| Combination 8 | sgRNA-2 + ssODN_8 | Combination 23 | sgRNA-5 + ssODN_19 |
| Combination 9 | sgRNA-2 + ssODN_9 | Combination 24 | sgRNA-5 + ssODN_20 |
| Combination 10 | sgRNA-2 + ssODN_10 | Combination 25 | sgRNA-6 + ssODN_21 |
| Combination 11 | sgRNA-2 + ssODN_11 | Combination 26 | sgRNA-7 + ssODN_22 |
| Combination 12 | sgRNA-2 + ssODN_12 | Combination 27 | sgRNA-8 + ssODN_23 |
| Combination 13 | sgRNA-2 + ssODN_13 | Combination 28 | sgRNA-8 + ssODN_24 |
| Combination 14 | sgRNA-2 + ssODN_14 | Combination 29 | sgRNA-8 + ssODN_25 |
| Combination 15 | sgRNA-2 + ssODN_15 | Combination 30 | sgRNA-8 + ssODN_26 |

### Example 3: Co-transfection of ssODN and sgRNA-spCas9 vector into K562 cells by electroporation for gene editing

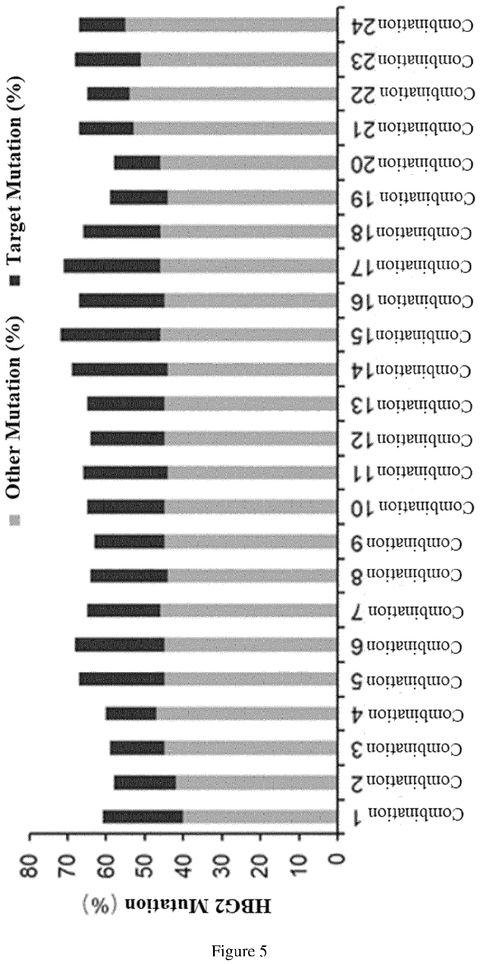

Transfection of plasmids into suspension cells using liposomes (e.g. Lipofectamine 2000^{™} reagent) is relatively inefficient, and transfecting suspension cells are generally introduced by electroporation. Some combinations of sgRNA and ssODN in Example 2 were taken to be introduced into K562 cells by lonza-4D electroporator, with the K562 cell transfection procedure which is built-in. The transfected cells were cultured for 4 days, and the cells were collected to extract genomic DNA. The fragment of HBG2 promoter gene was amplified by PCR to sequencing. The sequencing results analyzed by synthego software for gene editing efficiency, show that the electroporation of combinations sgRNA-1, sgRNA-2, sgRNA-5 and ssODN in K562 can achieve higher editing efficiency (Figure 5), which is slightly higher than that in 293T cells. And the expected mutation type induced by electroporation of ssODN and sgRNA-spCas9 vector was also slightly higher than that in 293T cells.

### Example 4: Co-transfection of ssODN and spCas9 protein/sgRNA RNP into K562 cells by electroporation for efficient gene editing

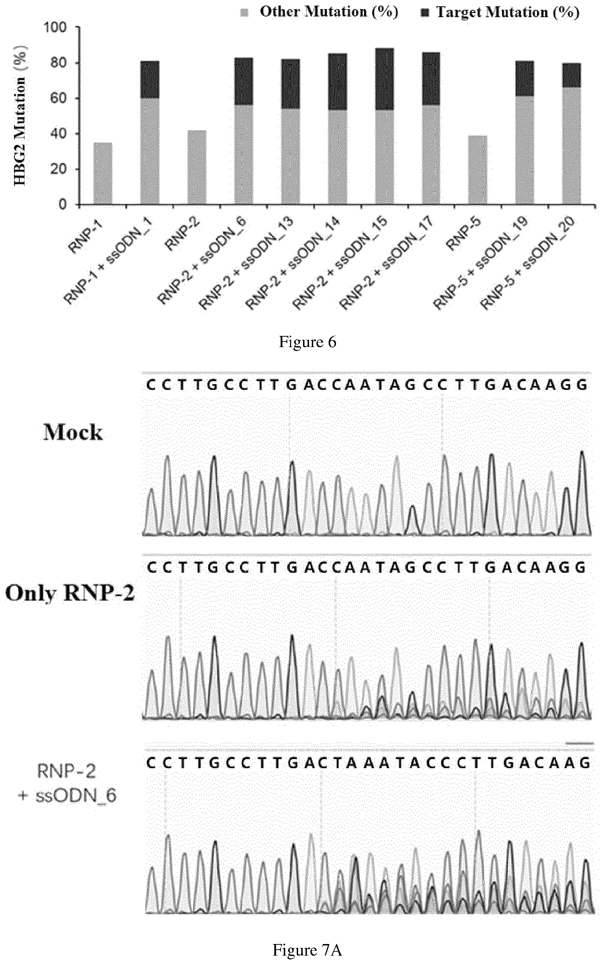

Compared with the plasmid-expressed CRISPR-Cas system, the delivery of the CRISPR-Cas system in the form of ribonucleoprotein (RNP) has been proved to have a high efficiency in gene editing and it is not easy to induce apoptosis. Commercial spCas9 protein and chemically synthesized sgRNA-1, sgRNA-2, and sgRNA-5 were used (sequences are the same as the corresponding sgRNAs in Table 4, with the terminus chemically modified) to incubate and package RNP-1, RNP-2 and RNP-5 *in vitro,* respectively. RNPs were delivered in combination with some ssODN into K562 cells by electroporation. The transfected cells were cultured for 4 days, and the cells were collected to extract genomic DNA. The HBG2 promoter gene fragment was amplified by PCR to conduct sequencing. The sequencing results analyzed by synthego software for gene editing efficiency, show that (Figure 6) compared with the combination without the addition of ssODN, the addition of ssODN when introducing RNP in K562 with electroporation significantly improved the overall gene editing efficiency by about two times (Figure 6). Meanwhile, transduction of ssODN and RNP by electroporation could induce a higher proportion of gene editing to form the expected mutation type (Figure 6).

### Example 5: Introduction of ssODN and spCas9 protein/sgRNA RNP into human hematopoietic stem/progenitor cells by electroporation for efficient gene editing and significantly increase HBG expression

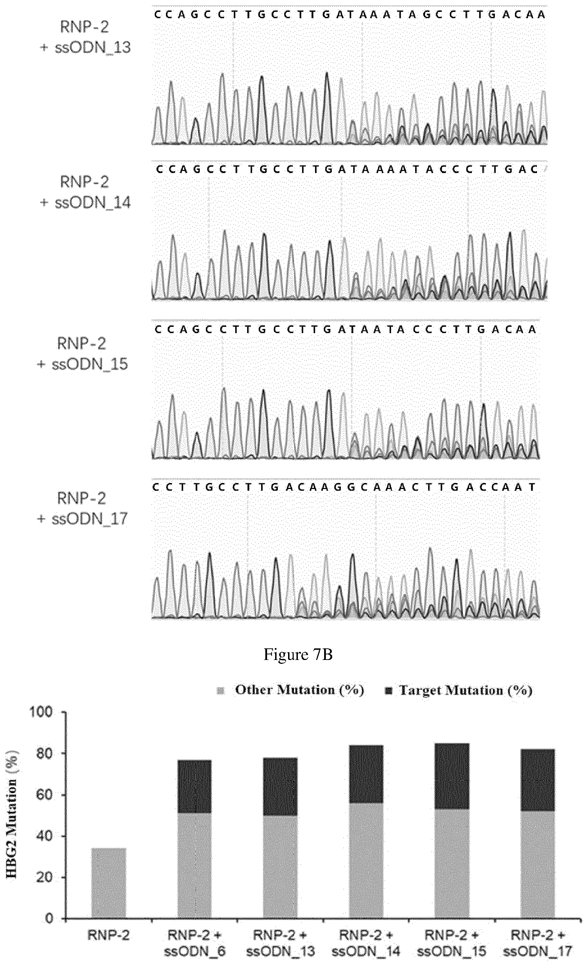

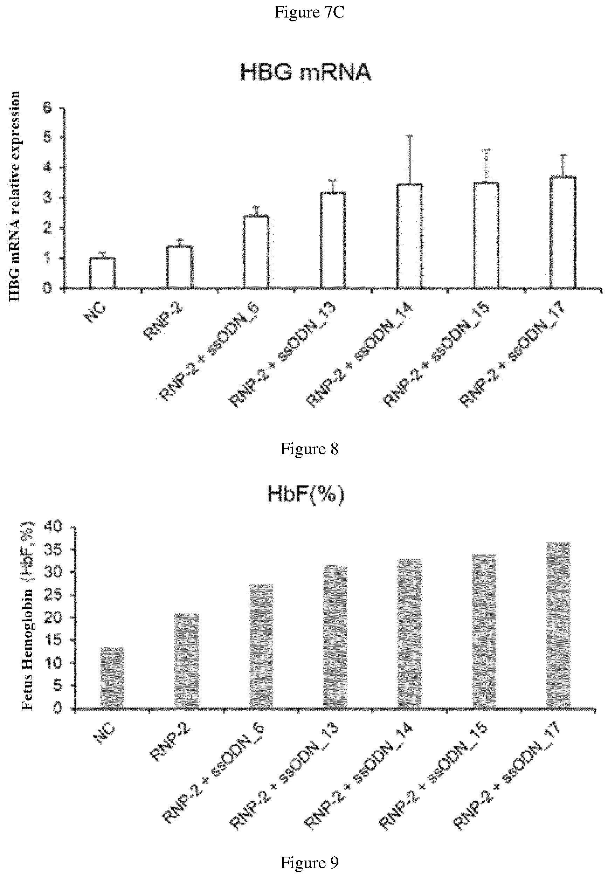

After resuscitation, mobilized peripheral blood-derived human CD34-positive cells (mPBSCs) were cultured for two days in StemSpan serum-free expansion medium (SFEM) enriched with human cytokines (SCF, TPO, Flt3L, 100 ng/ml each). ssODN and spCas9/sgRNA RNPs were delivered into mPBSCs by the EO-100 program built in the lonza-4D electroporator. After electroporation, the cells were cultured in SFEM enriched with human cytokines (SCF/TPO/Flt3L, 100 ng/ml each) for one day, and induced to differentiate erythrocytes in three stages. The first stage: culture in IMDM medium containing additives such as EPO, SCF, human AB serum, insulin, transferrin, heparin, IL-3, hydrocortisone, etc. for 7 days. On the 5th day of induced differentiation, 2 × 10e5 cells were taken out to extract genomic DNA, and then the HBG2 promoter fragment was amplified by PCR and sent to Sanger sequencing. Similar to the results in K562 cells, transduction of ssODN and spCas9/sgRNA RNPs by electroporation in mPBSCs resulted in efficient gene editing, with partial genome editing resulting in 30% of the expected mutation types (Figures 7A-7C).

Electroporated mPBSC cells and untreated mPBSC cells (control, NC) were induced for 7 days of the first stage of differentiation, and then were cultured in IMDM medium containing additives such as EPO, SCF, human AB serum, insulin, transferrin, and heparin, etc. for 4 days, and were finally cultured in IMDM medium containing additives such as EPO, human AB serum, insulin, transferrin, and heparin, etc. for 7 days. After 18 days of differentiation induction, both the electroporated mPBSCs and the untreated mPBSCs were observed dark red after centrifugation, which contained a large number of erythrocytes, indicating that the transfected mPBSCs with electroporation could differentiate into erythrocytes normally.

Near the end of differentiation induction, some cells were taken to extract mRNA, and the expression level of HBG mRNA was detected by reverse transcription-real-time quantitative PCR (RT-qPCR). Compared with non-transfected cells (NC), co-transfection of RNP-2 with ssODN_6, ssODN_13, ssODN_14, ssODN_15 and ssODN_17 all increased the expression level of HBG mRNA (Figure 8), and the increase of HBG mRNA could be more than 3-fold.

The glycosylated hemoglobin A1c detection system (VARIANT II TURBO HbAlc kit-2.0) was used to detect the hemoglobin of the erythrocytes differentiated from hematopoietic stem cells with electroporation transduction in the RNP-2 group by HPLC. The results showed that, compared with cells without gene editing, cells added with ssODN for gene editing has a significant increase in the content of fetal hemoglobin (HbF), ranging from 27% to 36%, which has more than 2-fold upregulation compared with the control group of 13.3%(Figure 9).

The results of the above embodiments show that the present disclosure provides a gene editing method for enhancing γ-globin gene expression that differs from the prior art. The ssODN disclosed in the present disclosure for γ-globin gene editing could guide the formation of the expected mutations at predetermined sites in the regulatory regions of HBG1 and HBG2 genes after gene editing, thereby forming an active GATA element. The element plays a positive regulatory role after the differentiation of erythrocytes from gene-edited cells, such as hematopoietic stem cells, activating or significantly increasing γ-globin gene expression. Therefore, the disclosure has potential application value in gene therapy of β-hemoglobinopathies.

The technical features of the embodiments described above can be combined randomly. Not all possible combinations of the technical features in the embodiments above are described for brevity. However, as long as there is no contradiction in the combinations of these technical features, they should be considered to be within the scope of the description.

The embodiments mentioned above only represent several embodiments of the present disclosure, and the descriptions thereof are specific and detailed, but should not be understood as a limitation on the scope of the present patent. It should be noted that for the skilled in the art, without departing from the concept of the present disclosure, modifications and improvements can also be made, which all belong to the protection scope of the present disclosure. Thus, the protection scope of the present disclosure should be subject to the appended claims.

## Claims

1. A method for activating γ-globin gene expression, comprising the following operations: a GATA or TATC sequence is artificially introduced into the sense strand or antisense strand of the regulatory region of γ-globin gene by gene editing technology, forming an enhancer element comprising a GATA motif in the regulatory region of γ-globin gene.

2. The method of claim 1, wherein the GATA or TATC sequence is introduced by deletion of the sequence, insertion of the sequence, mutation of the sequence, or a combination of the above.

3. The method of claim 1, wherein the GATA motif is a WGATAR sequence, or corresponding antisense complementary sequence YTATCW, wherein W is T or A, R is A or G, and Y is T or C.

4. The method of claim 1, wherein said enhancer element further comprises an NTG motif or an NAG motif, or corresponding antisense complementary sequence thereof, wherein N is A, G, C or T.

5. The method of claim 4, wherein the distance between said GATA motif and the NTG motif or NAG motif is 7 to 8 bases, and said NTG motif or NAG motif is located upstream from said GATA motif sequence.

6. An ssODN for gene editing, comprising a GATA or TATC sequence.

7. The ssODN of claim 6, wherein the ssODN comprises a 5' homology arm, a substitution sequence, and a 3' homology arm.

8. The ssODN of claim 7, wherein the 5' homology arm and the 3' homology arm of the ssODN are symmetric or asymmetric, and the length of the 5' homology arm and the 3' homology arm are 20 to 300 nt.

9. The ssODN of claim 7, wherein the 5' homology arm or the 3' homology arm of the ssODN is selected from the sense strand or the antisense strand of the intended-editing region.

10. The ssODN of claim 7, wherein the base number of the substitution sequence in the ssODN is 0-6.

11. The ssODN of claim 7, wherein the 3' terminus of the 5' homology arm is 0-20 bases apart from the 5' terminus of the 3' homology arm in the ssODN.

12. The ssODN of claim 6, wherein the ssODN comprises the sequence selected from the group consisting of SEQ ID NO: 4 to SEQ ID NO: 7, SEQ ID NO: 9 to SEQ ID NO: 22, SEQ ID NO: 24, SEQ ID NO: 26, SEQ ID NO: 28 to SEQ ID NO: 31, and the corresponding antisense complementary sequence thereof.

13. The ssODN of claim 6, wherein the ssODN comprises the sequence selected from the group consisting of SEQ ID NO: 40 to SEQ ID NO: 65, and the corresponding antisense complementary sequence thereof.

14. The ssODN of claim 6, wherein the ssODN is chemically modified with the 5' and 3' terminus are phosphorothioate-modified nucleotides.

15. An sgRNA comprising a guide sequence partially or fully complementary to the sense or antisense strand within the regulatory region of HBG1 or HBG2.

16. The sgRNA of claim 15, wherein the sequence of the regulatory region of HBG1 is shown in SEQ ID NO: 1.

17. The sgRNA of claim 15, wherein the sequence of the regulatory region of HBG2 is shown in SEQ ID NO: 2.

18. The sgRNA of claim 15, wherein the DNA sequence corresponding to the guide sequence of the sgRNA molecule is partially or completely the same as the sequence selected from the group consisting of SEQ ID NO: 32 to SEQ ID NO: 39.

19. The sgRNA of claim 15, wherein the sgRNA is any one or more sequence selected from the group consisting of SEQ ID NO: 69 to SEQ ID NO: 76.

20. A composition for gene editing comprising the ssODN of any one of claims 6 to 14, and a gene editing system targeting the region of HBG1 or HBG2.

21. The composition of claim 20, wherein the gene editing system is a CRISPR-Cas editing system, a TALEN editing system, a ZFN editing system.

22. The composition of claim 21, wherein the CRISPR-Cas editing system is a CRISPR-Cas9 system, or a CRISPR-Cas12 system.

23. The composition of claim 21, wherein the CRISPR-Cas editing system in the composition is a guide RNA and Cas protein.

24. The composition of claim 21, wherein the CRISPR-Cas editing system in the composition is an RNA complex composed of mRNA encoding Cas protein and gRNA.

25. The composition of claim 21, wherein the CRISPR-Cas editing system in the composition is a plasmid expressing Cas protein and gRNA.

26. The composition of claim 20, wherein the composition comprises the sgRNA of any one of claims 15 to 19, and the sgRNA is chemically synthesized or transcribed *in vitro.*

27. A kit for activating γ-globin gene, comprising:
(1) the ssODN of any one of claims 6-14;
(2) the sgRNA of any one of claims 15-19;
(3) At least one of a vector expressing Cas9 or Cas 12 protein, Cas9 or Cas 12 protein, and an mRNA corresponding to a Cas9 or Cas12 protein.

28. A hematopoietic stem cell obtained by transferring the composition of any one of claims 20 to 26 into a hematopoietic stem cell by electroporation.

29. A use of the ssODN of any one of claims 6-14, the sgRNA of any one of claims 15-19, the composition of any one of claims 20-26, the kit of claim 27, or the hematopoietic stem cell of claim 28 in the preparation of a cell or medicament for the treatment of β-thalassemia or sickle cell anemia.


Sequence Listing

<110>　GUANGZHOU REFORGENE MEDICINE CO.,LTD.

<120>　METHOD FOR ACTIVATING EXPRESSION OF GAMMA-GLOBIN GENE, AND COMPOSITION

<130>　2020

<160>　79

<170>　PatentIn version 3.3

<210>　1
<211>　1500
<212>　DNA
<213>　Homo sapiens

<400>　1
tctttctggc cttttagcca tctgtatcaa tgagcagata taagctttac acaggatcat　　60
gaaggatgaa agaatttcac caatattata ataatttcaa tcaacctgat agcttagggg　　120
ataaactaat ttgaagatac agcttgcctc cgataagcca gaattccaga gcttctggca　　180
ttataatcta gcaaggttag agatcatgga tcactttcag agaaaaacaa aaacaaacta　　240
accaaaagca aaacagaacc aaaaaaccac cataaatact tcctaccctg ttaatggtcc　　300
aatatgtcag aaacagcact gtgttagaaa taaagctgtc taaagtacac taatattcga　　360
gttataatag tgtgtggact attagtcaat aaaaacaacc cttgcctctt tagagttgtt　　420
ttccatgtac acgcacatct tatgtcttag agtaagattc cctgagaagt gaacctagca　　480
tttatacaag ataattaatt ctaatccaca gtacctgcca aagaacattc taccatcatc　　540
tttactgagc atagaagagc tacgccaaaa ccctgggtca tcagccagca cacacactta　　600
tccagtggta aatacacatc atctggtgta tacatacata cctgaatatg gaatcaaata　　660
tttttctaag atgaaacagt catgatttat ttcaaatagg tacggataag tagatattga　　720
ggtaagcatt aggtcttata ttatgtaaca ctaatctatt actgcgctga aactgtggct　　780
ttatagaaat tgttttcact gcactattga gaaattaaga gataatggca aaagtcacaa　　840
agagtatatt caaaaagaag tatagcactt tttccttaga aaccactgct aactgaaaga　　900
gactaagatt tgtcccgtca aaaatcctgg acctatgcct aaaacacatt tcacaatccc　　960
tgaacttttc aaaaattggt acatgcttta gctttaaact acaggcctca ctggagctag　　1020
agacaagaag gtaaaaaacg gctgacaaaa gaagtcctgg tatcctctat gatgggagaa　　1080
ggaaactagc taaagggaag aataaattag agaaaaactg gaatgactga atcggaacaa　　1140
ggcaaaggct ataaaaaaaa ttagcagtat cctcttgggg gccccttccc cacactatct　　1200
caatgcaaat atctgtctga aacggtccct ggctaaactc cacccatggg ttggccagcc　　1260
ttgccttgac caatagcctt gacaaggcaa acttgaccaa tagtcttaga gtatccagtg　　1320
aggccagggg ccggcggctg gctagggatg aagaataaaa ggaagcaccc ttcagcagtt　　1380
ccacacactc gcttctggaa cgtctgaggt tatcaataag ctcctagtcc agacgccatg　　1440
ggtcatttca cagaggagga caaggctact atcacaagcc tgtggggcaa ggtgaatgtg　　1500

<210>　2
<211>　1500
<212>　DNA

<213>    Homo sapiens

<400>    2
atctttctgg tcttttagcc gcctaacact ttgagcagat ataagcctta cacaggatta          60
tgaagtctga aaggattcca ccaatattat tataattcct atcaacctga taggttaggg         120
gaaggtagag ctctcctcca ataagccaga tttccagagt ttctgacgtc ataatctacc         180
aaggtcatgg atcgagttca gagaaaaaac aaaagcaaaa ccaaacctac caaaaaataa         240
aaatcccaaa gaaaaaataa agaaaaaaac agcatgaata cttcctgcca tgttaagtgg         300
ccaatatgtc agaaacagca ctgagttaca gataaagatg tctaaactac agtgacatcc         360
cagctgtcac agtgtgtgga ctattagtca ataaaacagt ccctgcctct taagagttgt         420
tttccatgca aatacatgtc ttatgtctta gaataagatt ccctaagaag tgaacctagc         480
atttatacaa gataattaat tctaatccat agtatctggt aaagagcatt ctaccatcat         540
ctttaccgag catagaagag ctacaccaaa accctgggtc atcagccagc acatacactt         600
atccagtgat aaatacacat catcgggtgc ctacatacat acctgaatat aaaaaaaata         660
cttttgctga gatgaaacag gcgtgattta tttcaaatag gtacggataa gtagatattg         720
aagtaaggat tcagtcttat attatattac ataacattaa tctattcctg cactgaaact         780
gttgctttat aggatttttc actacactaa tgagaactta agagataatg gcctaaaacc         840
acagagagta tattcaaaga taagtatagc acttcttatt tggaaaccaa tgcttactaa         900
atgagactaa gacgtgtccc atcaaaaatc ctggacctat gcctaaaaca catttcacaa         960
tccctgaact tttcaaaaat tggtacatgc tttaacttta aactacaggc ctcactggag        1020
ctacagacaa gaaggtgaaa aacggctgac aaaagaagtc ctggtatctt ctatggtggg        1080
agaagaaaac tagctaaagg gaagaataaa ttagagaaaa attggaatga ctgaatcgga        1140
acaaggcaaa ggctataaaa aaaattaagc agcagtatcc tcttgggggc cccttcccca        1200
cactatctca atgcaaatat ctgtctgaaa cggtccctgg ctaaactcca cccatgggtt        1260
ggccagcctt gccttgacca atagccttga caaggcaaac ttgaccaata gtcttagagt        1320
atccagtgag gccaggggcc ggcggctggc tagggatgaa gaataaaagg aagcaccctt        1380
cagcagttcc acacactcgc ttctggaacg tctgaggtta tcaataagct cctagtccag        1440
acgccatggg tcatttcaca gaggaggaca aggctactat cacaagcctg tggggcaagg        1500

<210>    3
<211>    27
<212>    DNA
<213>    Homo sapiens

<400>    3
ttgaccaata gtcttagagt atccagt                                              27

<210>    4
<211>    27
<212>    DNA
<213>    Artificial Sequence

<400>    4
ttgaccaata gtgatagagt atccagt                                              27

<210> 5
<211> 27
<212> DNA
<213> Artificial Sequence
<400> 5
ttgaccaata gagatagagt atccagt 27

<210> 6
<211> 26
<212> DNA
<213> Artificial Sequence

<400> 6
ttgaccaata agatagagta tccagt 26

<210> 7
<211> 26
<212> DNA
<213> Artificial Sequence

<400> 7
ttgaccaata tgatagagta tccagt 26

<210> 8
<211> 32
<212> DNA
<213> Homo sapiens

<400> 8
cagccttgcc ttgaccaata gccttgacaa gg 32

<210> 9
<211> 32
<212> DNA
<213> Artificial Sequence

<400> 9
cagccttgcc ttgacagata gccttgacaa gg 32

<210> 10

<211>    32
<212>    DNA
<213>    Artificial Sequence

<400>    10
cagccttgcc ttgactgata gccttgacaa gg                                        32



<210>    11
<211>    32
<212>    DNA
<213>    Artificial Sequence

<400>    11
cagccttgcc ttgagagata gccttgacaa gg                                        32




<210>    12
<211>    32
<212>    DNA
<213>    Artificial Sequence

<400>    12
cagccttgcc ttgagtgata gccttgacaa gg                                        32




<210>    13
<211>    32
<212>    DNA
<213>    Artificial Sequence

<400>    13
cagccttgcc ttgaaagata gccttgacaa gg                                        32
<210>    14
<211>    32
<212>    DNA
<213>    Artificial Sequence

<400>    14
cagccttgcc ttgaatgata gccttgacaa gg                                        32




<210>    15
<211>    32
<212>    DNA
<213>    Artificial Sequence

<400> 15
cagccttgcc ttgatagata gccttgacaa gg 32

<210> 16
<211> 32
<212> DNA
<213> Artificial Sequence
<400> 16
cagccttgcc ttgattgata gccttgacaa gg 32

<210> 17
<211> 32
<212> DNA
<213> Artificial Sequence

<400> 17
cagccttgcc ttgataaata gccttgacaa gg 32

<210> 18
<211> 33
<212> DNA
<213> Artificial Sequence
<400> 18
cagccttgcc ttgataaaat agccttgaca agg 33

<210> 19
<211> 31
<212> DNA
<213> Artificial Sequence

<400> 19
cagccttgcc ttgataatag ccttgacaag g 31

<210> 20
<211> 28
<212> DNA
<213> Artificial Sequence

<400> 20
cagccttgcc ttgatagcct tgacaagg 28

<210> 21
<211> 19
<212> DNA
<213> Artificial Sequence

<400> 21
cagccttgcc ttgataagg 19

<210> 22
<211> 20
<212> DNA
<213> Artificial Sequence

<400> 22
cagccttgcc ttgataaagg 20

<210> 23
<211> 23
<212> DNA
<213> Homo sapiens
<400> 23
tatctgtctg aaacggtccc tgg 23

<210> 24
<211> 29
<212> DNA
<213> Artificial Sequence

<400> 24
tatctgtctg aaacggtaga taaccctgg 29

<210> 25
<211> 33
<212> DNA
<213> Homo sapiens

<400> 25
cactatctca atgcaaatat ctgtctgaaa cgg 33

<210> 26
<211> 25
<212> DNA
<213> Artificial Sequence

<400> 26
cactatctca atgcaacaga aacgg 25

<210> 27
<211> 23
<212> DNA
<213> Homo sapiens
<400> 27
tccctgaact tttcaaaaat tgg 23

<210> 28
<211> 24
<212> DNA
<213> Artificial Sequence
<400> 28
tccctgaact tttcagataa ttgg 24

<210> 29
<211> 25
<212> DNA
<213> Artificial Sequence

<400> 29
tccctgaact tttcagataa attgg 25

<210> 30
<211> 26
<212> DNA
<213> Artificial Sequence

<400> 30
tccctgaact tttcagataa aattgg 26

<210> 31

<211> 26
<212> DNA
<213> Artificial Sequence

<400> 31
tccctgaact tttcagatag aattgg 26

<210> 32
<211> 23
<212> DNA
<213> Homo sapiens

<400> 32
actggatact ctaagactat tgg 23

<210> 33
<211> 23
<212> DNA
<213> Homo sapiens

<400> 33
cttgtcaagg ctattggtca agg 23

<210> 34
<211> 23
<212> DNA
<213> Homo sapiens

<400> 34
gtttgccttg tcaaggctat tgg 23

<210> 35
<211> 23
<212> DNA
<213> Homo sapiens

<400> 35
tggtcaagtt tgccttgtca agg 23

<210> 36
<211> 23

<212>  DNA
<213>  Homo sapiens

<400>  36
cttgaccaat agccttgaca agg                                                23

<210>  37
<211>  23
<212>  DNA
<213>  Homo sapiens

<400>  37
tatctgtctg aaacggtccc tgg                                                23

<210>  38
<211>  23
<212>  DNA
<213>  Homo sapiens

<400>  38
atgcaaatat ctgtctgaaa cgg                                                23

<210>  39
<211>  23
<212>  DNA
<213>  Homo sapiens

<400>  39
tccctgaact tttcaaaaat tgg                                                23

<210>  40
<211>  84
<212>  DNA
<213>  Artificial Sequence

<400>  40
cctagccagc cgccggcccc tggcctcact ggatactcta tcactattgg tcaagtttgc        60
cttgtcaagg ctattggtca aggc                                               84

<210>  41

<211>    84
<212>    DNA
<213>    Artificial Sequence

<400>    41
cctagccagc cgccggcccc tggcctcact ggatactcta tctctattgg tcaagtttgc    60
cttgtcaagg ctattggtca aggc    84

<210>    42
<211>    83
<212>    DNA
<213>    Artificial Sequence

<400>    42
cctagccagc cgccggcccc tggcctcact ggatactcta tcttattggt caagtttgcc    60
ttgtcaaggc tattggtcaa ggc    83

<210>    43
<211>    83
<212>    DNA
<213>    Artificial Sequence

<400>    43
cctagccagc cgccggcccc tggcctcact ggatactcta tcatattggt caagtttgcc    60
ttgtcaaggc tattggtcaa ggc    83

<210>    44
<211>    82
<212>    DNA
<213>    Artificial Sequence

<400>    44
ctctaagact attggtcaag tttgccttgt caaggctatc tgtcaaggca aggctggcca    60
acccatgggt ggagtttagc ca    82

<210>    45
<211>    82
<212>    DNA
<213>    Artificial Sequence

<400>    45
ctctaagact attggtcaag tttgccttgt caaggctatc agtcaaggca aggctggcca    60

acccatgggt ggagtttagc ca 82

<210> 46
<211> 82
<212> DNA
<213> Artificial Sequence

<400> 46
ctctaagact attggtcaag tttgccttgt caaggctatc tctcaaggca aggctggcca 60
acccatgggt ggagtttagc ca 82

<210> 47
<211> 82
<212> DNA
<213> Artificial Sequence

<400> 47
ctctaagact attggtcaag tttgccttgt caaggctatc actcaaggca aggctggcca 60
acccatgggt ggagtttagc ca 82

<210> 48
<211> 82
<212> DNA
<213> Artificial Sequence

<400> 48
ctctaagact attggtcaag tttgccttgt caaggctatc tttcaaggca aggctggcca 60
acccatgggt ggagtttagc ca 82

<210> 49
<211> 82
<212> DNA
<213> Artificial Sequence

<400> 49
ctctaagact attggtcaag tttgccttgt caaggctatc attcaaggca aggctggcca 60
acccatgggt ggagtttagc ca 82

<210> 50
<211> 82

<212>   DNA
<213>   Artificial Sequence

<400>   50
ctctaagact attggtcaag tttgccttgt caaggctatc tatcaaggca aggctggcca   60
acccatgggt ggagtttagc ca   82

<210>   51
<211>   82
<212>   DNA
<213>   Artificial Sequence

<400>   51
ctctaagact attggtcaag tttgccttgt caaggctatc aatcaaggca aggctggcca   60
acccatgggt ggagtttagc ca   82

<210>   52
<211>   82
<212>   DNA
<213>   Artificial Sequence

<400>   52
ctctaagact attggtcaag tttgccttgt caaggctatt tatcaaggca aggctggcca   60
acccatgggt ggagtttagc ca   82

<210>   53
<211>   83
<212>   DNA
<213>   Artificial Sequence

<400>   53
ctctaagact attggtcaag tttgccttgt caaggctatt ttatcaaggc aaggctggcc   60
aacccatggg tggagtttag cca   83

<210>   54
<211>   81
<212>   DNA
<213>   Artificial Sequence

<400>   54
ctctaagact attggtcaag tttgccttgt caaggctatt atcaaggcaa ggctggccaa   60
cccatgggtg gagtttagcc a   81

<210> 55
<211> 78
<212> DNA
<213> Artificial Sequence

<400> 55
ctctaagact attggtcaag tttgccttgt caaggctatc aaggcaaggc tggccaaccc 60
atgggtggag tttagcca 78

<210> 56
<211> 81
<212> DNA
<213> Artificial Sequence

<400> 56
cctcactgga tactctaaga ctattggtca agtttgcctt atcaaggcaa ggctggccaa 60
cccatgggtg gagtttagcc a 81

<210> 57
<211> 82
<212> DNA
<213> Artificial Sequence

<400> 57
cctcactgga tactctaaga ctattggtca agtttgcctt tatcaaggca aggctggcca 60
acccatgggt ggagtttagc ca 82

<210> 58
<211> 81
<212> DNA
<213> Artificial Sequence

<400> 58
tggctaaact ccacccatgg gttggccagc cttgccttga taaggcaaac ttgaccaata 60
gtcttagagt atccagtgag g 81

<210> 59
<211> 82
<212> DNA
<213> Artificial Sequence

<400>  59
tggctaaact ccacccatgg gttggccagc cttgccttga taaaggcaaa cttgaccaat  60
agtcttagag tatccagtga gg  82

<210>  60
<211>  86
<212>  DNA
<213>  Artificial Sequence

<400>  60
tccccacact atctcaatgc aaatatctgt ctgaaacggt agataaccct ggctaaactc  60
cacccatggg ttggccagcc ttgcct  86

<210>  61
<211>  84
<212>  DNA
<213>  Artificial Sequence

<400>  61
tatcctcttg ggggcccctt ccccacacta tctcaatgca acagaaacgg tccctggcta  61
aactccaccc atgggttggc cagc  84

<210>  62
<211>  83
<212>  DNA
<213>  Artificial Sequence

<400>  62
cctatgccta aaacacattt cacaatccct gaacttttca gataattggt acatgcttta  60
actttaaact acaggcctca ctg  83

<210>  63
<211>  84
<212>  DNA
<213>  Artificial Sequence

<400>  63
cctatgccta aaacacattt cacaatccct gaacttttca gataaattgg tacatgcttt  60
aactttaaac tacaggcctc actg  84

<210>  64
<211>  85

<212>  DNA
<213>  Artificial Sequence

<400>  64
cctatgccta aaacacattt cacaatccct gaacttttca gataaaattg gtacatgctt    60
taactttaaa ctacaggcct cactg                                          85

<210>  65
<211>  85
<212>  DNA
<213>  Artificial Sequence

<400>  65
cctatgccta aaacacattt cacaatccct gaacttttca gatagaattg gtacatgctt    60
taactttaaa ctacaggcct cactg                                          85

<210>  66
<211>  80
<212>  RNA
<213>  Artificial Sequence

<400>  66
guuuuagagc uagaaauagc aaguuaaaau aaggcuaguc cguuaucaac uugaaaaagu    60
ggcaccgagu cggugcuuuu                                                80

<210>  67
<211>  24
<212>  DNA
<213>  Artificial Sequence

<400>  67
ggactatcat atgcttaccg taac                                           24

<210>  68
<211>  20
<212>  DNA
<213>  Artificial Sequence

<400>  68
ggcgggccat ttaccgtaag                                                24

<210> 69
<211> 100
<212> RNA
<213> Artificial Sequence

<400> 69
acuggauacu cuaagacuau guuuuagagc uagaaauagc aaguuaaaau aaggcuaguc 60
cguuaucaac uugaaaaagu ggcaccgagu cggugcuuuu 100

<210> 70
<211> 100
<212> RNA
<213> Artificial Sequence

<400> 70
cuugucaagg cuauugguca guuuuagagc uagaaauagc aaguuaaaau aaggcuaguc 60
cguuaucaac uugaaaaagu ggcaccgagu cggugcuuuu 100

<210> 71
<211> 100
<212> RNA
<213> Artificial Sequence

<400> 71
guuugccuug ucaaggcuau guuuuagagc uagaaauagc aaguuaaaau aaggcuaguc 60
cguuaucaac uugaaaaagu ggcaccgagu cggugcuuuu 100

<210> 72
<211> 100
<212> RNA
<213> Artificial Sequence

<400> 72
uggucaaguu ugccuuguca guuuuagagc uagaaauagc aaguuaaaau aaggcuaguc 60
cguuaucaac uugaaaaagu ggcaccgagu cggugcuuuu 100

<210> 73
<211> 100
<212> RNA
<213> Artificial Sequence

<400> 73
cuugaccaau agccuugaca guuuuagagc uagaaauagc aaguuaaaau aaggcuaguc 60

cguuaucaac uugaaaaagu ggcaccgagu cggugcuuuu 100

<210> 74
<211> 100
<212> RNA
<213> Artificial Sequence

<400> 74
uaucugucug aaacgguccc guuuuagagc uagaaauagc aaguuaaaau aaggcuaguc 60
cguuaucaac uugaaaaagu ggcaccgagu cggugcuuuu 100

<210> 75
<211> 100
<212> RNA
<213> Artificial Sequence

<400> 75
augcaaauau cugucugaaa guuuuagagc uagaaauagc aaguuaaaau aaggcuaguc 60
cguuaucaac uugaaaaagu ggcaccgagu cggugcuuuu 100

<210> 76
<211> 100
<212> RNA
<213> Artificial Sequence

<400> 76
ucccugaacu uuucaaaaau guuuuagagc uagaaauagc aaguuaaaau aaggcuaguc 60
cguuaucaac uugaaaaagu ggcaccgagu cggugcuuuu 100

<210> 77
<211> 22
<212> DNA
<213> Homo sapiens

<400> 77
tactgcgctg aaactgtggc tt 22

<210> 78
<211> 22
<212> DNA
<213> Homo sapiens

<400> 78
cttcccaggg tttctcctcc ag 22

<210> 79
<211> 26
<212> DNA
<213> Homo sapiens

<400> 79

tgcactgaaa ctgttgcttt atagga 26